# EUROPÄISCHE PATENTSCHRIFT

(11) **EP 0 480 440 B1**
(45) Veröffentlichungstag und Bekanntmachung des Hinweises auf die Patenterteilung: **01.05.1996**
(21) Anmeldenummer: 91117323.5
(22) Anmeldetag: 10.10.1991
(51) Int. Cl.: C12P 21/08, A61K 39/395, G01N 33/577

(54) **Monoklonale Antikörper gegen Melanom**
Monoclonal antibody against melanoma
Anticorps monoclonal contre le mélanome

(30) Priorität: 11.10.1990 DE 4032312; 06.03.1991 DE 4107154
(43) Veröffentlichungstag der Anmeldung: 15.04.1992
(73) Patentinhaber: BOEHRINGER MANNHEIM GMBH, D-68298 Mannheim (DE)
(72) Erfinder: Brandt, Michael, Dr., W-8127 Iffeldorf (DE); Endl, Josef, Dr., W-8120 Weilheim (DE); Jungfer, Herbert, Dr., W-8130 Starnberg (DE); Albert, Winfried, Dr., W-8121 Eberfing (DE)
(74) Vertreter: Huber, Bernhard, Dipl.-Chem.

(56) Entgegenhaltungen:
- EP-A- 0 280 209
- EP-A- 0 316 882

## Beschreibung

Die vorliegende Erfindung betrifft ein Verfahren zur Gewinnung von humanen Antikörpern, die gegen Melanom gerichtet sind, die durch das erfindungsgemäße Verfahren erhaltenen Antikörper sowie deren Verwendung.

Melanom, ein Tumor auf der Haut, ist ein äußerst aggressiver Tumor. Insbesondere metastasierende Melanome sind mit konventionellen Methoden kaum mehr erfolgreich zu behandeln. Es ist daher ein großes Bedürfnis, neue Therapeutika zu finden, die für eine Behandlung von Melanomen geeignet sind.

Dippold et al. (Proc.Natl.Acad.Sci. USA 77 (1980), 6114-6118) berichteten über die Herstellung von murinen monoklonalen Antikörpern gegen Melanom. Einer der dort offenbarten Antikörper ist gegen das Gangliosid GD3 gerichtet. In J. Biol. Chem. 262 (1987), 6802-6807 sind ebenfalls murine monoklonale Antikörper gegen Melanom beschrieben. Diese binden gleichermaßen an GM3 und GM2, aber nicht an GD3.

In der EP-A-0 316 882 wird über die Herstellung von murinen monoklonalen Antikörpern berichtet, die spezifisch für ein Sialinsäure enthaltendes Glycolipid sind, welches einen N-Acetyl-Neuraminsäurerest mit einer alpha-2 → 3-Bindung trägt. Zu dieser Gruppe gehören u.a. GD3, GM3, GD2, GM2, GM1 und GD1a. Diese monoklonalen Antikörper reagieren mit Melanom. Eine Spezifität auf Melanom wird nicht offenbart.

1982 berichteten Irie et al. (Proc.Natl.Acad.Sci. USA 79, 5666-5670) über die Herstellung humaner monoklonaler Antikörper, die mit Melanom reagieren. Die dort offenbarten monoklonalen Antikörper reagieren mit den Gangliosiden GM2 bzw. GD2. In Proc.Natl.Acad.Sci. USA 84 (1987), 2416-2420 sind ebenfalls humane monoklonale Antikörper gegen Melanom beschrieben. Diese binden stark an GD3 und GD2 bzw. GM3 und GD1a. In Cancer Research 49 (1989), 191-196 sind ebenfalls humane monoklonale Antikörper gegen Melanom beschrieben. Diese binden stark an GM3 und GD1a bzw. GD3 und GD2. Die zur Herstellung dieser monoklonalen Antikörper verwendeten Lymphozyten stammen vom Melanom-Patienten.

Diese in der Literatur beschriebenen monoklonalen Antikörper wurden entweder von Melanom-Patienten mit oder ohne Immunisierung oder durch und nach Immunisierung von Labortieren erhalten. Derartige Versuchsansätze zur Gewinnung von auch in vivo gegen Melanom wirksamen Antikörpern weisen jedoch große Nachteile auf. Murine monoklonale Antikörper haben den Nachteil, daß sie vom humanen Immunsystem als Fremdproteine erkannt werden und daher Antikörper gegen diese Fremdproteine gebildet werden. Dies bedeutet, daß solche monoklonalen Antikörper schneller eliminiert werden und damit in ihrer Wirksamkeit eingeschränkt sind. Aber auch bei Antikörpern, die aus Tumor-Patienten gewonnen wurden, bestehen große Zweifel an ihrer Wirksamkeit, da gerade bei Tumor-Patienten die Funktionsfähigkeit des Immunsystems nicht gegeben ist. Überdies erkennen die Antikörper des Standes der Technik bei weitem nicht alle primären Melanome und auch nur einen geringen Anteil von Melanom-Metastasen.

Aufgabe der vorliegenden Erfindung war es daher, Antikörper gegen Melanom bereitzustellen, bei denen die Nachteile des Standes der Technik mindestens teilweise beseitigt sind.

Die erfindungsgemäße Aufgabe wird gelöst durch Bereitstellung eines Antikörpers gegen Melanom, der dadurch gekennzeichnet ist, daß er an die Ganglioside GM3 und GD3 bindet, im wesentlichen aber nicht an die Ganglioside GM1, GM2, GD1a, GD1b und GD2 bindet, wobei die Bindung des Antikörpers an die Ganglioside durch Immunfärbung nach dünnschichtchromatographischer Auftrennung der Ganglioside bestimmt wurde.

Die erfindungsgemäßen Antikörper, bei denen es sich um humane Antikörper handelt, sind erhältlich durch ein Verfahren, wobei man ohne vorherige Immunisierung B-Lymphozyten aus einer gesunden Person isoliert, die isolierten B-Lymphozyten immortalisiert, Antikörper aus den immortalisierten B-Lymphozyten durch immunhistochemische Analyse auf Bindung gegen primäres Melanom oder/und Melanom-Metastasen untersucht, die positiv-reagierenden Klone auswählt, kultiviert und daraus monoklonale Antikörper gewinnt.

Die Gewinnung von gegen Melanom gerichteten Antikörpern aus gesunden Personen, die nicht an Melanom erkrankt sind, ist völlig überraschend. Vorzugsweise wählt man als Donor für die B-Lymphozyten eine gesunde Melanom-Risikoperson aus. Unter einer Melanom-Risikoperson ist z.B. ein Mensch mit einem Sonnenbrandrisiko zu verstehen, d.h. ein hellhaariger oder hellhäutiger Mensch, gegebenenfalls mit Sommersprossen, der zuvor (besonders bevorzugt über einen längeren Zeitraum hinweg) einer intensiven UV-Bestrahlung ausgesetzt war. Die Eignung des erfindungsgemäßen Verfahrens zur Gewinnung von humanen, gegen Melanom gerichteten und auch wirksamen Antikörpern läßt sich möglicherweise darauf zurückführen, daß anscheinend in jeder Person ständig Zellen entarten, wodurch Melanomzellen entstehen, die aber vom körpereigenen Immunsystem erfolgreich bekämpft werden können.

Der erste Schritt des erfindungsgemäßen Verfahrens ist die Gewinnung von B-Lymphozyten aus dem Blut einer gesunden Person. Eine "gesunde Person" im Sinne der vorliegenden Erfindung ist als Mensch definiert, der keine Symptome eines Melanoms zeigt. Dies kann nach an sich bekannten Methoden erfolgen. Anschließend erfolgt die Immortalisierung der Lymphozyten. Dafür stehen verschiedene Methoden zur Verfügung. Die B-Lymphozyten können mit Myelomzellen humaner oder muriner Herkunft nach der Methode von Köhler und Milstein (Nature 256 (1975), 495) fusioniert werden. Zur Fusion können aber auch Heteromyelome verwendet werden. Ebenso ist es möglich, die B-Lymphozyten mittels Epstein-Barr-Virus zu transformieren. Weiterhin kann das in der EP-A 0 093 436 oder das in der EP-A 0 256 512 beschriebene Verfahren verwendet werden. Dabei wird mit subzellulären Vesikeln, die eine transformierende DNA enthalten, fusioniert.

Von den immortalisierten B-Lymphozyten werden diejenigen ausgewählt, die Antikörper mit der gewünschten Reaktivität gegen Melanom produzieren. Die Selektion der B-Lymphozyten auf Sekretion von Antikörpern, die gegen Melanom gerichtet und auch gegen Metastasen wirksam sind, erfolgt erfindungsgemäß durch immunhistochemische Analyse auf Bindung gegen Melanom-Gewebeschnitte, wobei man primäres Melanom oder/und Melanom-Metastasen verwenden kann. Vorzugsweise wird die immunhistochemische Analyse der Antikörper durch Bindung an Melanom in Gewebeschnitten mittels eines ELISA-Tests in Anlehnung an die Methode von Nielsen et al. (Hybridoma 6 (1987), 103-109) untersucht. Auf diese Weise positiv ermittelte B-Lymphozyten werden ausgewählt, nach üblichen Verfahren gezüchtet und daraus nach dem Fachmann bekannten Methoden monoklonale Antikörper gewonnen.

Durch das erfindungsgemäße Verfahren ist es möglich, Antikörper gegen Melanom zu erhalten, die auch eine sehr hohe Reaktivität gegen Melanom-Metastasen besitzen. Somit beinhaltet die vorliegende Erfindung auch humane Antikörper gegen Melanom, die in Gewebeschnitten auch an mindestens 70 % der Melanom-Metastasen binden.

Ein erfindungsgemäßer Antikörper ist dadurch gekennzeichnet, daß er an die Ganglioside GM3 und GD3 bindet, im wesentlichen aber nicht an die Ganglioside GM1, GM2, GD1a, GD1b und GD2 bindet. Dies bedeutet, daß die erfindungsgemäßen Antikörper an die Ganglioside GM1, GM2, GD1a, GD1b und GD2 mit einer Affinität von höchstens etwa 5 % bezüglich der Affinität für das Gangliosid GM3 oder das Gangliosid GD3 binden. Die Bestimmung der Bindefähigkeit der erfindungsgemäßen Antikörper an die Ganglioside erfolgt durch Immunfärbung nach dünnschichtchromatographischer Auftrennung der Ganglioside. Durch dieses Verfahren ist es auf zuverlässige Weise möglich zu entscheiden, ob ein spezifisches Gangliosid an einen Antikörper bindet oder nicht. Weniger bevorzugt ist die Bestimmung der Affinität eines Antikörpers nach der Methode des ELISA-Tests. Bei dieser Methode können unspezifische Bindungen nicht ausgeschlossen werden, so daß die Möglichkeit von falsch-positiven Ergebnissen besteht. Diese falsch-positiven Ergebnisse würden sich in einer geringen Bindungsaffinität an Ganglioside äußern, bei denen nach dem anderen oben beschriebenen Verfahren keine Bindung festzustellen ist.

Beispiele für Antikörper, die mit Hilfe des erfindungsgemäßen Verfahrens gewonnen werden können, sind die monoklonalen Antikörper "17" und AH18, sekretiert von den Hybridomzellinien ECACC 90090703 und ECACC 90090701. Diese Antikörper sind von der Klasse IgM.

Ein weiterer Gegenstand der Erfindung sind humane, monoklonale Antikörper, die in äquivalenter Weise bindefähig sind wie die aus den Hybridomzellinien ECACC 90090703 oder ECACC 90090701 erhältlichen monoklonalen Antikörper. Unter dem Begriff "in äquivalenter Weise bindefähiger Antikörper" werden Antikörper verstanden, bei denen eine Epitopüberlappung mit dem in Betracht kommenden Antikörper nachweisbar ist. Diese Epitopüberlappung kann mit Hilfe eines kompetitiven Testsystems nachgewiesen werden. Dazu wird z.B. mit Hilfe eines Enzym-Immunoassays überprüft, inwieweit ein Antikörper mit dem bekannten Antikörper um die Bindung an ein definiertes Antigen bzw. ein spezielles Epitop konkurriert. Dazu inkubiert man ein entsprechendes immobilisiertes Antigen mit dem erfindungsgemäßen Antikörper in markierter Form und einem Überschuß des in Betracht gezogenen Antikörpers. Durch Nachweis der gebundenen Markierung kann dann leicht festgestellt werden, inwieweit der in Betracht gezogene Antikörper den definierten Antikörper aus der Bindung verdrängen kann. Ist eine Verdrängung von mindestens 50 % bei 10⁵-fachem Überschuß gegeben, so liegt eine Epitopüberlappung vor.

Überraschenderweise wurde auch festgestellt, daß erfindungsgemäße Antikörper nicht nur an Melanom, sondern auch an andere Tumorgewebe, insbesondere Lungen- und Brustkarzinomgewebe binden können. Ein Beispiel hierfür ist der erfindungsgemäße Antikörper "17".

Weiterhin beinhaltet die vorliegende Erfindung Derivate von erfindungsgemäßen Antikörpern, die deren Bindungsspezifität besitzen, jedoch Modifikationen in dem nicht für die Antigenbindung wesentlichen Bereich aufweisen. Diese Antikörperderivate können etwa aus den erfindungsgemäßen Antikörpern durch Austausch einer oder mehrerer konstanter Domänen oder/und Verknüpfung mit einem anderen Molekül erhalten werden. So kann z.B. ein Austausch von konstanten Domänen für einen Isotype-Switch durchgeführt werden, wo z.B. ein Antikörper der Klasse IgM in einen Antikörper der Klasse IgG unter Beibehaltung seiner Antigen-Spezifität überführt werden kann. Dieser Isotype-Switch kann durch zellbiologische oder molekularbiologische Methoden erfolgen, die dem Fachmann bekannt sind (z.B. Rothman P. et al., Mol.Cell.Biol. 10 (1990), 1672-1679). Der monoklonale Antikörper gemäß vorliegender Erfindung kann jedoch auch mit einem anderen Molekül, insbesondere einem Marker oder einem Toxin verknüpft werden, wodurch seine diagnostische oder therapeutische Verwendbarkeit verändert wird. Geeignete Verfahren zur Verknüpfung von Markern (z.B. Enzymen wie Peroxidase) oder Toxinen (z.B. Ricin, Choleratoxin) mit dem Antikörper bzw. die radioaktive Markierung sind dem Fachmann bekannt.

Ein weiterer Gegenstand der vorliegenden Erfindung ist die Verwendung eines erfindungsgemäßen Antikörpers zur Diagnose oder Therapie von Melanom, insbesondere zur passiven und aktiven Immunisierung von Melanompatienten. Vorzugsweise verwendet man dabei den von der Zellinie ECACC 90090703 sekretierten Antikörper "17" oder/und den von der Zellinie ECACC 90090701 sekretierten Antikörper AH18.

Da die nach dem erfindungsgemäßen Verfahren erhaltenen monoklonalen Antikörper an lebende Melanomzellen und andere Tumorzellen binden, können sie auch zur Bekämpfung dieser Zellen im Organismus eingesetzt werden. Somit beinhaltet die vorliegende Erfindung auch ein Arzneimittel, das aus einem oder mehreren erfindungsgemäßen Antikörpern, gegebenenfalls zusammen mit üblichen pharmazeutischen Träger-, Hilfs-, Füll- und Zusatzstoffen besteht. Die Verabreichung eines erfindungsgemäßen Arzneimittels ist sowohl zur Vorbeugung als auch nach einer Metastasierung eines Tumors, insbesondere eines Melanoms möglich. Eine zur passiven Immunisierung geeignete Dosis der erfindungsgemäßen Antikörper liegt im Bereich von 1 bis 200 mg, wobei diese Dosis gegebenenfalls wiederholt zu verabreichen ist. Die monoklonalen Antikörper können lokal in das Melanom, wie von Irie und Morton (Proc.Natl.Acad.Sci. USA 83 (1986), 8694-8698) beschrieben, verabreicht werden. Nach Metastasierung des Melanoms ist jedoch eine, dem Fachmann geläufige systemische Verabreichung bevorzugt. Die erfindungsgemäßen Antikörper werden vorzugsweise alleine therapeutisch eingesetzt, können jedoch auch als Konjugate mit Toxinen, Therapeutika und ähnlichem verwendet werden.

Da die nach dem erfindungsgemäßen Verfahren gewonnenen Antikörper mit Melanomen und Melanom-Metastasen bindefähig sind, eignen sie sich auch hervorragend zum qualitativen oder quantitativen Nachweis von Melanom- und anderen Tumorzellen im Gewebe. Der Nachweis erfolgt dabei in an sich bekannter Weise durch ein immunologisches Bestimmungsverfahren. Verfahren dieser Art sind dem Fachmann geläufig und brauchen daher hier nicht mehr erläutert zu werden. Der erfindungsgemäß gewonnene Antikörper kann dabei als unmarkierter, markierter oder/und immobilisierter Rezeptor eingesetzt werden.

Die durch den monoklonalen Antikörper definierten Antigene bzw. Epitope können ebenfalls durch immunologische Bestimmungsverfahren in Körperflüssigkeiten nachgewiesen werden. Die erfindungsgemäßen monoklonalen Antikörper können dabei als markierter oder/und immobilisierter Rezeptor eingesetzt werden. Für die Durchführung des Bestimmungsverfahrens sind viele Varianten bekannt, die alle geeignet sind. So können zwei oder drei oder auch mehr Rezeptoren verwendet werden und die Inkubation mit den einzelnen Rezeptoren kann in verschiedener Reihenfolge in homogener oder heterogener Phase erfolgen. Ausgewertet wird jeweils eine durch Bindung von mindestens zwei Rezeptoren mit der in der Probelösung nachzuweisenden Substanz erfolgende Signaländerung. Bevorzugt erfolgt die erfindungsgemäße Bestimmung entweder in homogener Phase, z.B. nach dem Prinzip des Agglutinationstests, wobei als Rezeptoren beschichtete Partikel wie z.B. Latex-Partikel oder Erythrozyten verwendet werden, die durch Bindung mit spezifisch bindefähigen Rezeptoren und der nachzuweisenden Zelle vernetzen und dadurch agglutinieren, oder in heterogener Phase, bevorzugt als Sandwich-Immunoassay. In jedem Falle werden mindestens zwei Rezeptoren R₁ und R₂ eingesetzt, von denen einer einen erfindungsgemäßen monoklonalen Antikörper (z.B. "17") oder einen in äquivalenter Weise bindefähigen Antikörper oder ein Derivat davon enthält, während der andere Rezeptor einen anderen erfindungsgemäßen Antikörper (z.B. AH18) oder einen in äquivalenter Weise bindefähigen Antikörper oder ein Derivat davon enthält.

Bei Inkubation der Körperflüssigkeit mit den beiden Rezeptoren bilden sich nun Komplexe aus R₁, Gangliosid und R₂. Die Rezeptoren werden so ausgesucht, daß nur Komplexe, in denen sowohl R₁ als auch R₂ mit dem Gangliosid verbunden sind, eine Signaländerung ergeben, so daß auf diese Weise nur solche Ganglioside erfaßt werden, die mit den beiden spezifischen Antikörpern bindefähig sind.

Bevorzugt erfolgt die erfindungsgemäße Bestimmung als Sandwich-Immunoassay. Dazu wird Rezeptor R₁ immobilisiert oder immobilisierbar gemacht und mit der Probelösung umgesetzt. Anschließend wird Rezeptor R₂ zugegeben. Es bilden sich Komplexe aus dem immobilisierten Rezeptor R₁ , dem nachzuweisenden Gangliosid und Rezeptor R₂.

Die erfindungsgemäßen Antikörper können auch zur Bestimmung eines prognostischen Index eingesetzt werden. Dabei wird nach einer Behandlung eines Melanom-Patienten festgestellt, ob die durch die monoklonalen Antikörper erkannten Antigene in den Körperflüssigkeiten, insbesondere im Serum des Patienten nach einer gewissen Zeit auftauchen oder verschwinden.

Schließlich beinhaltet die vorliegende Erfindung auch ein Verfahren zur in vitro Diagnose von Tumoren, insbesondere von Melanomen, wobei man einen oder ein Gemisch von mehreren erfindungsgemäßen Antikörpern, gegebenenfalls zusammen mit üblichen pharmazeutischen Träger-, Hilfs-, Füll- und Zusatzstoffen, vorzugsweise in einer Dosis von 1 bis 200 mg verabreicht.

Die in der Erfindung genannten Zellinien ECACC 90090703 und ECACC 90090701, welche die Antikörper "17" und AH18 sekretieren, wurden am 07. September 1990 bei der European Collection of Animal Cell Cultures, Porton Down (GB) hinterlegt.

Die Erfindung wird durch die folgenden Beispiele erläutert.

### Beispiel 1

### Selektion auf Antikörper gegen Melanom

Einer gesunden Person werden 20 bis 200 ml Blut abgenommen und daraus die mononukleären Zellen isoliert. Anschließend werden diese Zellen nach herkömmlichen Methoden immortalisiert (Köhler/Milstein bzw. EBV-Transformation bzw. DNA-Transfektion). Nach 2 bis 4 Wochen werden die Kulturüberstände der immortalisierten Zellen getestet. Dazu wird ein ELISA-Test an humanen Gewebeschnitten von Melanom durchgeführt. Dies geschieht in Anlehnung an die Methode von Nielsen et al. (Hybridoma 6 (1987), 103-109).

Es werden tiefgefrorene Tumorgewebe in 3 bis 5 µm dicke Scheiben geschnitten und auf mit Silan vorbehandelten Deckgläschen aufgebracht. Die Gewebeschnitte können bei -80°C gelagert oder auch gleich verwendet werden. Dazu werden sie nach Bedarf für 10 Minuten bei -20°C in Aceton fixiert. Anschließend läßt man gegebenenfalls das Aceton abdampfen und taucht danach die Schnitte für ca. 3 Minuten in PBS (phosphatgepufferte Salzlösung nach Dulbecco und Vogt, J.Exp.Med. 99 (1954), 167-182). Nach Abtropfen der PBS werden 20 bis 30 µl eines Blockierungsantikörpers (Konzentration 40 µg/ml) aufgebracht. Dieser Blockierungsantikörper ist das Fab-Fragment eines polyklonalen Schaf-Serums oder eines murinen monoklonalen Antikörpers gegen humanen Fcµ bzw. Fcγ. Der Blockierungsantikörper wird für 1 bis 2 Stunden oder über Nacht bei Raumtemperatur bzw. bei 4°C auf den Schnitten inkubiert.

Anschließend wird der Schnitt 3x ca. 4 Minuten lang in PBS bei 4°C gewaschen. Dann wird die zu untersuchende Antikörper-haltige Lösung (d.h. die Kulturüberstände der immortalisierten Zellen) aufpipettiert. Nach mehrstündiger (üblicherweise 2-stündiger) Inkubation bei 4°C wird die Antikörper-haltige Lösung durch dreimaliges ca. 4 Minuten dauerndes Waschen in PBS weggespült. Anschließend wird der Schnitt mit dem gleichen Antikörper wie bei der Blockierung inkubiert, nur daß diesmal der verwendete monoklonale bzw. polyklonale Antikörper an Peroxidase gekoppelt ist (2 U/ml). Es wird 1 Stunde bei 4°C inkubiert und anschließend, wie oben beschrieben, gewaschen. Dann werden die Objektträger in Substratlösung (Aminoethylcarbazol in DMSO/Tris HCl 50 mmol/l, pH 7,3) in Gegenwart von H₂O₂ eingetaucht und ca. 4 bis 5 Minuten inkubiert. Anschließend werden die Objektträger ca. 5 Minuten lang in PBS gewaschen.

Wenn gewünscht, können die Zellkerne in den Gewebeschnitten mit Hämalaun (Merck, 1:3 verdünnt in H₂O) gegengefärbt werden. Dazu werden die Objektträger für ca. 20 Sekunden in die Hämalaun-Lösung eingetaucht und anschließend in zwei PBS-Bädern (je ca. 5 Min.) "gebläut".

Abschließend werden die Schnitte mit wäßrigem Einbettmittel (z.B. Glycerin-Gelatin, Fa. Merck oder Krystal Mount, Fa. Biomeda Corp., Foster City, CA) zur Lagerung überschichtet.

Zellinien, welche die gewünschten Antikörper produzieren, werden expandiert und kloniert. Die erhaltenen Klone werden ebenfalls, wie oben beschrieben, analysiert. Die Klone, welche die gewünschten Antikörper produzieren, werden weitergezüchtet und die von diesen Klonen produzierten Antikörper nach bekannten Methoden gewonnen.

Durch dieses Verfahren konnten die erfindungsgemäßen Antikörper "17" und AH18 erhalten werden.

### Beispiel 2

### Antikörper-Spezifitätstest

Um die Spezifität der gemäß Beispiel 1 aus den immortalisierten Zellinien gewonnenen humanen monoklonalen Antikörpern zu ermitteln, wurde ein Test zur Bindung an humanes primäres Melanom und an humane Melanom-Metastasen durchgeführt. Die Durchführung des Tests erfolgte wie in Beispiel 1 beschrieben. Dabei wurden mit den erfindungsgemäßen Antikörpern "17" und AH18 die folgenden Ergebnisse erhalten.

**Tabelle 1:**

| humaner monoklonaler Antikörper | Nävi | Reaktivität mit | |
|---|---|---|---|
| | | Primäres Melanom (Zahl positiv/Zahl getestet) | Metastase |
| "17" | 28/29 | 48/52 (90%) | 54/54 (100%) |
| AH18 | 23/26 | 48/54 (90%) | 43/51 (80%) |

### Beispiel 3

### 3.1 Bindefähigkeit des Antikörpers an normales Gewebe

Der gemäß Beispiel 1 gewonnene monoklonale Antikörper "17" wurde auf seine Bindefähigkeit mit normalen Geweben getestet. Die Testdurchführung war wie in Beispiel 1.

Der Antikörper wurde auf Reaktivität gegen humanes Gewebe aus Gehirn (Cortex, Pons, Thalamus, Corpus amygdaloideum), Retina, Haut (Keratinozyten, Melanozyten, Langerhans-Zellen, Endothelial-Zellen, glatte Muskulatur, Schweißdrüsen, Nervenfasern), Muskel, Brust, Uterus, Harnblase, Gallenblase, Milz, Niere, Nebenniere, Lunge, Parotis, Darm, Erythrocyten (4 kommerzielle Testproben und 300 Blut-Donoren) und Nebenschilddrüse getestet. Es wurde keine Reaktion des erfindungsgemäßen Antikörpers mit den obigen Gewebearten gefunden.

### 3.2 Bindefähigkeit der Antikörper an Tumore.

Um die weitere Spezifität der gemäß Beispiel 1 aus den immortalisierten Zellinien gewonnenen humanen monoklonalen Antikörpern zu bestimmen, wurden weitere Tests an humanen Tumorgeweben durchgeführt. Die Durchführung des Tests erfolgte wie im Beispiel 1 beschrieben. Dabei wurden mit den erfindungsgemäßen Antikörpern die folgenden Ergebnisse erhalten:

**Tabelle 2**

| Tumor | Reaktivität mit "17" bzw. AH18 Zahl positive/Zahl getestete |
|---|---|
| Melanom | 48/52 |
| Lungenkarzinom | 10/19 |
| Brustkarzinom | 4/9 |

### Beispiel 4

Die Reaktivität der monoklonalen Antikörper gegen gereinigte Ganglioside wurde weiterhin in einem Immunoblot nach Dünnschichtchromatographie der Ganglioside untersucht.

Dafür wurden die Ganglioside GM3, GM2 und GD1a von Boehringer Mannheim, GD2 und GD3 von Biocarb, Schweden, GM1 von Fidia, Italien und GD1b von Pallmann, München verwandt. Die Dünnschichtplatten HPTLC Alu Kieselgel 60 F₂₅₄ wurden von Merck bezogen. Das Laufmittel für die Ganglioside bestand aus Chloroform: Methanol:H₂O (0.02% CaCl₂ x 2 H₂O) 60:40:9. Als Fixiermittel für die Dünnschichtplatten wurde Polyisobutylacrylat (high molecular weight), Aldrich Chemicals, als 0.1%-Lösung in Hexan eingesetzt.

### Durchführung:

Die Prozedur und Modifikationen davon sind dem Fachmann geläufig und werden hier nur beispielhaft wiedergegeben.

### Probenauftrag:

Es werden 5 µl einer 1 mg/ml Lösung der Ganglioside aufgetragen. Als Lösungsmittel wurde das oben genannte Laufmittel verwendet. Die Proben wurden z. B. mit einer Hamilton-Spritze auf einem ca. 5 mm breiten Streifen auf der Dünnschichtplatte sukzessive aufgetragen; man läßt dazwischen immer wieder gut antrocknen, damit der Strich möglichst dünn bleibt. Zum Schluß wird die Dünnschichtplatte nochmals gut mit einem Föhn getrocknet. Die Platte wird in eine mit Laufmittel gesättigte Kammer gegeben und man inkubiert, bis das Laufmittel etwa 80% der Platte angefeuchtet hat. Anschließend wird die Platte entnommen und man läßt sie trocknen.

Dann wird die Platte in 0.1%iger Polyisobutylacrylat-Lösung für etwa 1 Min geschwenkt.
Man läßt die Platte wiederum trocknen.
Zwecks Blockierung wird die Platte mittels einer Pipette mit einer 1% RSA/PBS (Rinder Serumalbumin, Boehringer Mannheim) Lösung bedeckt (keine Luftblasen lassen!) und ca. 30 Min bei Raumtemperatur stehen gelassen. Danach wird die Blockierungslösung abgegossen.
Anschließend wird 2x mit PBS gewaschen. Dazu wird die Platte in einer Schale mit PBS untergetaucht und ca. je 2 Min stehen gelassen. PBS wurde dazwischen abgesaugt (PBS nie direkt auf die Platte geben; nicht schütteln; Platte nicht austrocknen lassen).

Danach wird die Platte mit einer den monoklonalen Antikörper enthaltenden Lösung (Konzentration 1 - 10 µg/ml) überschichtet und für 1 Stunde bei Raumtemperatur stehen gelassen.

Anschließend wird 5x mit PBS gewaschen (wie oben beschrieben). Dann wird die Platte mit Konjugat (polyklonales, Peroxidase-markiertes Schaf-Fab-Fragment gegen humanes Fcµ, Konzentration wie beim Gewebetest Beispiel 1) überschichtet und für 1 Stunde bei Raumtemperatur inkubiert. Anschließend wird 6x mit PBS (wie oben beschrieben) gewaschen. Dann wird Substrat zugegeben und die Platte während der Entwicklung leicht geschwenkt.

Als Substrat wird TMB/DONS verwendet (Tetramethylbenzidin (TMB) 12 mg + Dioctylnatriumsulfosuccinat (DONS) 40 mg in 10 ml Methanol (bei 37°C) lösen und mit 10 ml Zitronensäure/Phosphat-Puffer pH 5.0 (25 ml 0.1 M Zitronensäure, Merck, + 28 ml 0.2 M Na₂HPO₄x2 H₂O, Merck, auf 100 ml) sowie mit 10 µl H₂O₂ (30%) mischen). Es wird solange entwickelt, wie die Negativkontrolle nicht gefärbt wird; dann wird mehrmals mit H₂O dest gewaschen. Die Platte wird lichtgeschützt getrocknet und sofort photographiert, da die Färbung mit TMB/DONS ausbleicht.

Führt man mit MAK 17 bzw. mit MAK AH 18 die oben beschriebene Prozedur durch, so erhält man die in Tabelle 3 gezeigten Ergebnisse. Die MAKs 17 und AH 18 reagieren mit GM 3 und GD 3, nicht aber mit GM 1, GM 2, GD 1a, GD 1b und GD 2.

Zur Kontrolle wurden die Ganglioside in einem Parallelversuch durch Anfärbung mit Resorcin sichtbar gemacht. Dazu wird die Platte mit Resorcin-Lösung (Resorcin von Merck, 400 ml + 100 ml H₂O + 5 ml H₂SO₄) eingesprüht und für etwa 10 Min im Trockenschrank bei 110 °C entwickelt.

Diese Kontrolle zeigt, daß von allen Gangliosiden gleiche Mengen auf die Dünnschicht-Platte aufgetragen wurden.

**Tabelle 3**

| **Nachweis der Ganglioside nach Dünnschichtchromatographie** | | |
|---|---|---|
| | Resorcin | MAK"17" bzw. MAK AH18 |
| GM1 | + | - |
| GM2 | + | - |
| GM3 | + | + |
| GD1a | + | - |
| GD1b | + | - |
| GD2 | + | - |
| GD3 | + | + |

Aus Tabelle 3 ist ersichtlich, daß die erfindungsgemäßen Antikörper nur mit bestimmten Gangliosiden reagieren. Von den getesteten Gangliosiden zeigen GM3 und GD3 positive Signale. Es wird keine nennenswerte Reaktivität (≤ 5 %) mit den Gangliosiden GM1, GM2, GD1a, GD2 und GD1b gefunden.

### Beispiel 5

### Reaktivität der Antikörper mit Zellinien

Zellen der zu untersuchenden Zellinien werden über Nacht in Terasaki-Platten (erhalten von Fa. Greiner) gezüchtet. Der Kulturüberstand wird von den adhärenten Zellen entfernt und durch die zu untersuchende Antikörperlösung ersetzt. Nach einer 1- bis 2-stündigen Inkubation wird die Antikörper-Lösung entfernt, der Zellrasen mehrmals gewaschen und der an die Zellen gebundene monoklonale Antikörper nachgewiesen. Dazu wurden nach dem Waschen mit PBS 100 µl Peroxidase-markierter Schaf-Anti-Human-Leichte-Kette-Antikörper zugegeben und wiederum für 1 bis 2 Stunden bei Raumtemperatur inkubiert. Nach erneutem Waschen wurde die Enzymreaktion mit Peroxidasesubstrat (ABTS®) gestartet. Nach 10 bis 60 Minuten bei Raumtemperatur wurde die Extinktion bei 406 nm in einem Photometer bestimmt. Alternativ dazu kann auch ein Peroxidasesubstrat wie Aminoethylcarbazol zugegeben werden und nach Beendigung der Reaktion der bräunliche Niederschlag an bzw. auf den Zellen mittels eines Mikroskops ausgewertet werden.

Aus dem in Tabelle 4 dargestellten Ergebnis geht hervor, daß der Antikörper 17 mit Melanom SK-MEL 28 Zellen (ATCC HTB 72) reagiert, während keine Reaktion mit humanen Vorhautfibroblasten gefunden wird (die nach bekannten Verfahren selbst isoliert wurden). Der Antikörper 17 zeigt weiterhin Reaktion mit Insulinom-Zellen RIN (erhalten von Dr. Eisenbart, Josslin Diabetes Center, Boston, Mass. 02215) und den Neuroblastom-Zellen IMR 32 (ATCC CCL 127).

**Tabelle 4:**

| Antikörper | Antikörper-Reaktivität gegen verschiedene Zellinien | | | |
|---|---|---|---|---|
| | SK MEL 28 (Melanom) | RIN (Insulinom) | IMR32 (Neuroblastom) | humane Vorhaut-Fibroblasten |
| "17" | ++ | + | + | - |

### Beispiel 6

### Bestimmung der Epitopüberlappung von Antiköpern gegen Melanom

Zum Nachweis der Epitopüberlappung eines Antikörpers mit einem der monoklonalen Antikörper ECACC 90090703 oder ECACC 90090701 wird ein kompetitiver Enzym Immunoassay durchgeführt. Dazu werden die von Boehringer Mannheim, Biocarb, Pallmann bzw. Fidia bezogenen Ganglioside GM3, GM2, GDla, GD2, GD3, GM1 und GD1b (vgl. Beispiel 4) in Methanol gelöst (10 µg/ml) und jeweils 100 µl dieser Lösung in 96-er Mikrotiterplatten (Greiner) pipettiert. Nach Eindampfen der Lösung (entweder über Nacht bei Raumtemperatur oder während 1 Stunde bei 37°C) wird mit PBS gewaschen und dann unspezifische Bindungsstellen mit 1 %-iger Crotein C-Lösung in PBS blockiert (Inkubation bei Raumtemperatur für 1-2 Stunden und Waschen mit PBS/0,05 % Tween 20). Anschließend wird 90 Min. bei Raumtemperatur simultan mit einem der monoklonalen Antikörper ECACC 90090703 oder ECACC 90090701, der mit Peroxidase markiert wurde (Endkonzentration 250 mU/ml), und dem zu beurteilenden Antikörper inkubiert. Nach erneutem 4-maligen Waschen mit PBS/0,05 % Tween 20 wird mit der Enzymsubstratlösung ABTS® in Natriumperborat enthaltendem Puffer 30 Min. bei Raumtemperatur inkubiert und anschließend die Extinktion bei 405 nm als Maß für die Menge des gebundenen, POD-markierten monoklonalen Antikörpers ECACC 90090703 oder ECACC 90090701 gemessen. Dieser Wert wird mit der Extinktion verglichen, die bei Inkubation mit dem monoklonalen Antikörper ECACC 90090703 oder ECACC 90090701 alleine erhalten wird (unter Zugabe einer entsprechenden Menge Puffer zum Ausgleich des bei Zugabe des zu beurteilenden Antikörpers auftretenden Verdünnungseffekts). Wenn bis zu einem 10⁵-fachen Überschuß an zu beurteilendem Antikörper gegenüber dem monoklonalen Antikörper ECACC 90090703 oder ECACC 90090701 Enzymkonjugat (250 mU/ml) mindestens 50 % Kompetition zu erkennen sind, liegt eine Epitopüberlappung vor.

## Patentansprüche

1. Humaner, monoklonaler Antikörper gegen Melanom,
**dadurch gekennzeichnet,**
daß er an die Ganglioside GM3 und GD3 bindet und die Ganglioside GM1, GM2, GD1a, GD1b und GD2 mit einer Affinität von höchstens 5% bezüglich der Affinität für das Gangliosid GM3 oder das Gangliosid GD3 bindet, wobei die Bindung des Antikörpers an die Ganglioside durch Immunfärbung nach dünnschichtchromatographischer Auftrennung der Ganglioside bestimmt wurde.

2. Antikörper nach Anspruch 1,
**dadurch gekennzeichnet**,
daß er ein Antikörper der Klasse IgM ist.

3. Antikörper nach einem der Ansprüche 1 bis 2,
**dadurch gekennzeichnet**,
daß er auch an mindestens 70 % der Melanom-Metastasen bindet.

4. Antikörper nach einem der Ansprüche 1 bis 3,
**dadurch gekennzeichnet**,
daß er zusätzlich an Lungenkarzinome oder/und Brustkarzinome binden kann.

5. Humaner, monoklonaler Antikörper nach Anspruch 1, erhältlich aus den Hybridomzellinien ECACC 90090703 oder ECACC 90090701.

6. Verfahren zur Herstellung von gegen Melanom gerichteten, humanen, monoklonalen Antikörpern nach einem der Ansprüche 1 bis 5,
**dadurch gekennzeichnet**,
daß man ohne vorherige Immunisierung B-Lymphozyten aus einer gesunden Person isoliert, die isolierten B-Lymphozyten immortalisiert, Antikörper aus den immortalisierten B-Lymphozyten durch immunhistochemische Analyse auf Bindung gegen primäres Melanom oder/und Melanom-Metastasen untersucht, die positiv reagierenden B-Lymphozyten-Klone auswählt, kultiviert und daraus monoklonale Antikörper gewinnt.

7. Derivat eines Antikörpers nach einem der Ansprüche 1 bis 5,
**dadurch gekennzeichnet,**
daß es sich von dem Antikörper
(1) durch Austausch von nicht für die Bindungsspezifität des Antikörpers verantwortlichen Domänen unterscheidet oder/und
(2) mit einem anderen Molekül verknüpft ist.

8. Verwendung eines Antikörpers oder Antikörperderivats nach einem der Ansprüche 1 bis 5 oder 7 zur in vitro Diagnose von Tumoren, insbesondere Melanomen.

9. Verwendung nach Anspruch 8, worin man einen von den Zellinien ECACC 90090703 oder ECACC 90090701 sekretierten Antikörper einsetzt.

10. Pharmazeutische Zusammensetzung,
**dadurch gekennzeichnet**,
daß sie als Wirkstoff einen oder mehrere Antikörper oder Antikörperderivate nach einem der Ansprüche 1 bis 5 oder 7, gegebenenfalls zusammen mit üblichen pharmazeutischen Träger-, Hilfs-, Füll- und Zusatzstoffen enthält.

11. Verwendung eines oder mehrerer Antikörper oder Antikörperderivate nach einem der Ansprüche 1 bis 5 oder 7, gegebenenfalls zusammen mit üblichen pharmazeutischen Träger-, Hilfs-, Füll- und Zusatzstoffen zur Herstellung eines Mittels für die Anwendung in einem Verfahren zur Diagnose oder Therapie von Tumoren, insbesondere Melanomen.

## Claims

1. Human monoclonal antibody against melanoma, characterised in that it binds to the gangliosides GM3 and GD3 and binds the gangliosides GM1, GM2, GD1a, GD1b and GD2 with an affinity of at most 5% with regard to the affinity for the ganglioside GM3 or the ganglioside GD3, whereby the binding of the antibody to the gangliosides was determined by immune staining after thin layer chromatographic separation of the gangliosides.

2. Antibody according to claim 1, characterised in that it is an antibody of class IgM.

3. Antibody according to one of claims 1 to 2, characterised in that it also binds to at least 70% of the melanoma metastases.

4. Antibody according to one of claims 1 to 3, characterised in that it can additionally bind to lung carcinoma and/or mammary carcinoma.

5. Human monoclonal antibody according to claim 1, obtainable from the hybridoma cell lines ECACC 90090703 or ECACC 90090701.

6. Process for the production of human monoclonal antibodies directed against melanoma according to one of claims 1 to 5, characterised in that, without previous immunisation, one isolates B-lymphocytes from a healthy person, immortalises the isolated B-lymphocytes, screens antibodies from the immortalised B-lymphocytes by immune-histochemical analysis for binding against primary melanoma and/or melanoma metastases, selects the positively reacting B-lymphocytes, cultures and obtains monoclonal antibodies therefrom.

7. Derivative of an antibody according to one of claims 1 to 5, characterised in that it differs from the antibody
(1) by exchange of domains not responsible for the binding specificity of the antibody and/or
(2) is connected with another molecule.

8. Use of an antibody or antibody derivative according to one of claims 1 to 5 or 7 for the in vitro diagnosis of tumours, especially of melanomas.

9. Use according to claim 8, wherein one uses an antibody secreted by the cell line ECACC 90090703 or ECACC 90090701.

10. Pharmaceutical composition, characterised in that, as active material, it contains one or more antibodies or antibody derivatives according to one of claims 1 to 5 or 7, possibly together with usual pharmaceutical carrier, adjuvant, filling and additive materials.

11. Use of one or more antibodies or antibody derivatives according to one of claims 1 to 5 or 7, possibly together with usual pharmaceutical carrier, adjuvant, filling and additive materials, for the production of an agent for use in a process for the diagnosis or therapy of tumours, especially of melanomas.

## Revendications

1. Anticorps monoclonal humain anti-mélanome, caractérisé en ce qu'il se fixe sur les gangliosides GM3 et GD3, et en ce qu'il se fixe sur les gangliosides GM1, GM2, GD1a, GD1b et GD2 avec une affinité qui représente au plus 5 % de l'affinité pour le ganglioside GM3 ou le ganglioside GD3, la fixation de l'anticorps sur les gangliosides ayant été déterminée par immunocoloration après une séparation des gangliosides par chromatographie en couche mince.

2. Anticorps selon la revendication 1, caractérisé en ce qu'il s'agit d'un anticorps de la classe des IgM.

3. Anticorps selon l'une des revendications 1 et 2, caractérisé en ce qu'il se fixe aussi sur au moins 70 % des métastases de mélanome.

4. Anticorps selon l'une des revendications 1 à 3, caractérisé en ce qu'il peut se fixer en outre sur le cancer du poumon ou/et le cancer du sein.

5. Anticorps monoclonal humain selon la revendication 1, pouvant être obtenu à partir des lignées cellulaires d'hybridomes ECACC 90090703 ou ECACC 90090701.

6. Procédé de production d'anticorps monoclonaux humains dirigés contre le mélanome selon l'une des revendications 1 à 5, caractérisé en ce que l'on isole des lymphocytes B d'une personne saine sans immunisation préalable, on immortalise les lymphocytes B isolés, on examine par analyse immunohistochimique les anticorps produits par les lymphocytes B immortalisés quant à la liaison à un mélanome primaire ou/et à des métastases de mélanome, on sélectionne les clones de lymphocytes B à réaction positive, on les cultive et on en isole les anticorps.

7. Dérivé d'un anticorps selon l'une des revendications 1 à 5, caractérisé en ce que
(1) il se distingue de l'anticorps par l'échange de domaines non responsables de la spécificité de la liaison de l'anticorps, ou/et
(2) il est relié à une autre molécule.

8. Utilisation d'un anticorps ou d'un dérivé d'anticorps selon l'une des revendications 1 à 5 ou 7 pour le diagnostic in vitro de tumeurs, en particulier de mélanomes.

9. Utilisation selon la revendication 8, dans laquelle on utilise un anticorps sécrété par les lignées cellulaires ECACC 90090703 ou ECACC 90090701.

10. Préparation pharmaceutique, caractérisée en ce qu'elle contient comme substance active un ou plusieurs anticorps ou dérivés d'anticorps selon l'une des revendications 1 à 5 ou 7, éventuellement avec des supports, agents auxiliaires, charges et additifs pharmaceutiques classiques.

11. Utilisation d'un ou plusieurs anticorps ou dérivés d'anticorps selon l'une des revendications 1 à 5 ou 7, éventuellement avec des supports, agents auxiliaires, charges et additifs pharmaceutiques classiques, pour la préparation d'une composition à utiliser dans un procédé de diagnostic ou de thérapie de tumeurs, en particulier de mélanomes.
